# EUROPEAN PATENT APPLICATION

(11) **EP 3 085 774 A1**
(43) Date of publication of application: **26.10.2016**
(21) Application number: 15164197.4
(22) Date of filing: 20.04.2015
(51) Int. Cl.: C12N 7/02

(54) **METHOD FOR CONCENTRATING, PURIFYING AND/OR ISOLATING VIRUS PARTICLES**

(71) Applicant: QIAGEN GmbH, 40724 Hilden (DE)
(72) Inventor: Singer, Thorsten, 42699 Solingen (DE); Kubicek, Johann, 50933 Köln (DE); Sander, Antje-Kathrin, 46569 Hünxe (DE)
(74) Representative: f & e patent

(57) **Abstract**

The present invention relates to a method for concentrating, purifying and/or isolating virus particles from a sample comprising said virus particles and optionally non-viral proteins including a step of crosslinking the proteinaceous portion of the virus particles, the use of a crosslinker for concentrating, purifying and/or isolating virus particles from a sample as well as to a kit for concentrating, purifying and/or isolating virus particles.

## Description

The present invention relates to a method for concentrating, purifying and/or isolating virus particles from a sample comprising said virus particles and optionally non-viral proteins including a step of crosslinking the proteinaceous portion of the virus particles, the use of a crosslinker for concentrating, purifying and/or isolating virus particles from a sample as well as to a kit for concentrating, purifying and/or isolating virus particles.

In the state of the art several methods are known for isolating or purifying virus particles, including various gradients and ultracentrifugation methods are known in the state of the art. In addition, methods based on ultrafiltration or diafiltration are known. Filtration-based methods are, however, not suitable for isolating virus particles from blood samples, including blood plasma, due to the high protein content in these samples which lead to clogging of the filtering unit.

Conventional virus purification methods based on sucrose or caesium chloride gradient (ultra)centrifugation and non-gradient ultracentrifugation are tedious, time-consuming and require expensive instrumentation. A typical non-gradient ultracentrifugation method e.g. requires spinning the sample in a centrifuge at a force of typically at least 40,000 xg for at least several hours.

Chromatographic methods on the other hand, such as e.g. ion-exchange chromatography, size exclusion chromatography or liquid affinity chromatography on the other hand require specifically adapted columns and further chromatographic equipment.

It was therefore an object of the present invention to provide a method for concentrating, purifying and/or isolating virus particles from a sample which avoids the above-mentioned drawbacks of the state of the art. Such a method in particular should allow to separate the virus particles in an essentially solid form from the majority of the liquid part of the sample by a simple physical separation process, preferably avoiding the need for ultracentrifugation and chromatographic methods. In addition, such a method should allow the subsequent isolation and/or analysis of nucleic acids from the virus particles, if desired.

This object is met by the method and the kit of the present invention.

The present invention provides a method for concentrating, purifying and/or isolating virus particles from a sample comprising said virus particles and optionally non-viral proteins, comprising the steps of:
i) providing an essentially liquid sample comprising said virus particles,
ii) mixing said sample with at least one crosslinker to crosslink the virus particles with each other via the proteinaceous portion of the virus particles and/or to crosslink the virus particles with non-viral proteins, if present in the sample,
iii) optionally incubating the mixture obtained in step ii),
iv) essentially separating the crosslinked virus particles from the remaining liquid part of the sample.

The crosslinker or the mixture of crosslinkers added to the sample reacts with functional groups in the side chains of any proteins present in the sample, crosslinking said proteins to further side chains in other protein molecules, leading to a network of crosslinked protein structures with a molecular weight high enough to be pelleted by standard centrifugation and forces of less than 40,000 xg or obtainable by filtration. As virus particles comprise a protein shell (a capside) enclosing and protecting the genetic material of the virus, said proteinaceous portion of the virus particle will be crosslinked to other virus particles and/or further non-viral proteins, as present in the sample, to form a high molecular weight network which can be separated from the remaining liquid part of the sample.

In terms of the present invention the term "non-viral proteins" includes any protein which might be present in the sample and do not represent a part of the protein shell of a virus, including e.g. extracellular proteins and former intracellular proteins as well as former membrane proteins, including integral, peripheral and liquid-anchored membrane proteins of prokaryotic and eukaryotic origin, including bacteria, archae, fungi, plant and animal proteins, preferably extracellular proteins of the aforementioned origin, more preferably mammal extracellular proteins as e.g. found in blood plasma, including human serum albumine (HAS) and immunoglobulins.

The term "former intracellular proteins and membrane proteins" refers to proteins which were comprised in cells contained in the sample before said cells were disrupted or separated from the sample to obtain the essentially liquid sample. Preferably the majority, i.e. at least 75 wt.-% of any cells originally present in the initial sample are physically removed from the sample, e.g. by centrifugation, or are disrupted by cell lysis, e.g. by mechanical or chemical methods to obtain the essentially liquid sample. If different types of cells have been present in the sample, e.g. microbial cells, including bacterial, archae and fungi cells or a combination thereof in the presence of higher eukaryotic cells, preferably animal, more preferably mammalian cells, it may be preferred to selectively lyse the higher eukaryotic cells, while leaving said microbial cells essentially intact. Said selective lysis of the higher eukaryotic cells may either be carried out in the presence of the virus particles, i.e. before physically separating the remaining microbial cells from the virus particles and before crosslinking the virus particles in the remaining sample, or after separating the mixture of microbial and higher eukaryotic cells from that part of the sample, which comprises the virus particles.

In terms of the present invention the "essentially liquid sample" comprising said virus particle comprises any sample comprising virus particles and more than 50 vol.-%, preferably at least 60 vol.-%, more preferably at least 70 vol.-%, even more preferably at least 75 vol.-%, 80 vol.-%, 85 vol.-%, or 90 vol.-%, and most preferably at least 95 vol.-% of a liquid. Said liquid may be selected from water, aqueous solutions, buffer solutions, cell culture media, naturally occurring body fluids or a mixture thereof, without being limited to the mentioned. It may also be possible to dilute a naturally occurring sample, e.g. with water or a suitable buffer, before adding the at least one crosslinker.

Preferably the sample may comprise or may be derived from tissue, tissue homogenates, cell cultures, bone marrow aspirates, bone homogenates, swabs, swab rinsates or body fluids, preferably body fluids, more preferably body fluids being selected from the group comprising amniotic fluid, aqueous humor, bile, bladder lavage, blood, breast exudates, bronchoalveolar lavage, cerebrospinal fluid, chyle, chyme, cytosol, feces, gastric contents, interstitial fluid, joint fluid, lymph, menses, mucus, plasma, pleural fluid, pus, saliva, sebum, semen, serum, sputum, sweat, synovial fluid, tears, urine, vaginal secretions and vitreous humour.

As already explained above it may be preferred to remove and/or disrupt cellular material present in the initial sample, e.g. by means of centrifuging, filtering and/or decanting or by cell lysis and subsequent removal of remaining cell debris from the liquid part of the sample. Preferably the essentially liquid sample of step i) may be obtained from an initial sample, comprising animal cells and optionally at least one further type of cells, selected from microbial and plant cells or a combination thereof, by a method comprising the steps of:
a) essentially selectively lysing the animal cells substantially without lysing the further type of cells, if present, and without digesting the proteinaceous portion (capside) of the virus particles by contacting the initial sample with an essentially protease- and chaotrope-free lysis solution A, and
b) essentially removing any remaining cells and/or cell debris from the liquid part of the sample, preferably by means of centrifuging, filtering and/or decanting.

A suitable lysis solution A will be described in detail below.

The term "essentially" shall be understood in a way that in any case more than 50% of the considered trait is obtained, preferably at least 60 %, more preferably at least 70 %, even more preferably at least 75 %, 80 %, 85 %, 90 %, or 95 % and most preferably at least 98 %. "Essentially" can also represent 100%. The term "substantially" shall be understood in similar way.

The term "to be derived from" is used to indicate that the sample as taken from its original biological environment may have been processed e.g. by homogenization, dilution, concentration, preservation, lysis and the like, without being limited to these.

The term "animal cells" shall comprise in particular mammal cells, but further insect cells, bird cells, poultry cells, fish cells, amphibian cells, reptilian cells, molluscan cells, wherein mammal cells are preferred and human cells are particularly preferred.

For crosslinking at least the proteinaceous portion of the virus particles with each other and/or with non-viral proteins, as present in the sample, the sample is mixed with at least one crosslinker. A mixture of different crosslinkers may be used as well. The crosslinker(s) may be added to the sample as a solution of the crosslinker(s) in any suitable solvent or mixture of solvents, e.g. water, or may be added in form of a compound, i.e. in an undissolved or undiluted form, to be at least partially diluted and/or dissolved by the liquid present in the sample.

Mixing of the sample with the crosslinker(s) may be supported by the usual measures known in the state of the art for this purpose, including for example shaking, stirring, swirling, pipetting up and down and the like, without being limited to these.

In an optional additional step the mixture thus obtained may be incubated for a certain time while no active mixing of the sample is carried out (not including physical mixing processes such as for example mixing by diffusion and the like). As an alternative, the sample may be incubated in presence of the crosslinker under permanent mixing, i.e. steps (ii) and (iii) may be carried out simultaneously. It may also be possible to actively mix the sample in contact with the crosslinker for certain periods of time, each of these mixing periods interrupted by incubation periods, wherein no active mixing is carried out.

Regardless whether steps (ii) and (iii) are carried out simultaneously, in succession or by turns, steps (ii) and (iii) together preferably may last from about 10 s to about 12 h, preferably from 15 s to about 10 h, more preferably from 30 s to about 5 h, even more preferably from 1 min to 3 h, still even more preferably from 2.5 min to 1.5 h and most preferably from 5 min to 1 h. If these steps are carried out as at least partially separated steps, both of them independently may be carried out at a temperature in the range of from -5°to 50°C, preferably from 0° to 40 °C, even more preferably from 5° to 30 °C and most preferably from 10°C to room temperature (20°C ± 5°C). If both steps are carried out as one combined step said combined step preferably may be carried out at a temperature in the same range.

By the formed crosslinks the virus particles are at least partially connected to each other or to non-virus proteins, thus, resulting in a network of virus particles and proteins, which is no longer finely dispersed or solved. The solid portion of the sample comprising the crosslinked virus particles is then allowed or forced to settle/separate from the liquid part of the sample and the solid part of the sample is essentially separated from the remaining liquid part. This may be carried out by means of centrifuging, filtering, allowing to settle due to gravitation and the like, and then removing the solid part from the remaining liquid part or vice versa by e.g. decanting, filtering, aspirating, pipetting or the like, without being limited to these.

In terms of the present invention crosslinking refers to the chemical connection of two or more functional groups, preferably functional groups from different molecules, by covalent bonds. Crosslinkers useful in the present inventive method are molecules with particular functional groups reactive with functional groups present in the side chains of amino acids, i.e. proteins. Functional groups present in the side chains of amino acids (enhanced protein) include amino groups, thiols, carboxyl groups, carboxyamido groups, hydroxyl groups, without being limited to these, which is well known to a person skilled in the art. By using crosslinkers with functional groups reactive with these groups present in side chains of amino acids, crosslinking of proteins can be achieved.

The method of the present invention furthermore may comprise at least one of the following steps:
v) essentially removing the crosslinks in the solid part of the sample obtained in step iv) to obtain free virus particles, and/or
vi) lysing the virus particles to release their nucleic acids into solution for further isolation, purification and/or analysis

It should be understood that either step v) nor step vi) may be carried out without carrying out the other step or that both steps may be carried out in the method of the present invention. Removal of the crosslinks in the solid part of the sample may either be obtained by removing at least one of the at least two bonds one crosslinker molecule introduced into the proteinaceous portion of the virus particles and/or with a non-viral protein, if present in step ii) or by cleaving a bond inside the crosslinker molecule which is not immediately linked to any protein molecule. Which way of removing the crosslinks is preferred depends on the process samples and the crosslinker(s) used. If e.g. a crosslinker having at least one carbonyl group as the functional group reactive with amino groups in the side chains of protein was used, it may be preferred to cleave the amino groups formed between said aldehyde and said amino group, e.g. by hydrolysis. If on the other hand carbodiimide reactive groups were used in the crosslinker to react with amine or carboxyl groups in the proteins it may be preferred to cleave an internal bond of the crosslinker for removing the crosslinks, e.g. an internal disulfide bond.

The particular conditions for removing the crosslinks in the solid part depend on the sample and the crosslinker used, however, removal of crosslinking usually may be achieved at a temperature in the range of from 20 to 100°C.. Intermal disulfide bonds e.g. may conveniently be cleaved by adding dithiotritol (DTP) to the crosslinked sample while imino bonds formed by the reaction of an aldehyde-based crosslinker with an amino group of a protein may e.g. be cleaved by adding a tris buffer to the sample.

Lysis of the virus particles to release their nucleic acids into the solution for further isolation, purification and/or analysis may be carried out using any method known in the state of the art for this purpose.

For instance, kits for lysing virus particles and isolating their nucleic acids are commercially available under the trademark names QIAamp Min Elute Virus Spin (QIAGEN Hilden, Germany), Nucleus Spin, RNA virus (Macherey Nagel, Düren, Germany), without being limited to these. In principle, any kit and/or method known in the state of the art for lysing the capside of virus particles and subsequently isolating, purifying and/or analyzing the least nucleic acid may be used.

Step iv) of essentially separating the solid part of the sample comprising crosslinked virus particles from the remaining liquid part preferably may be achieved by spinning the sample in a centrifuge at a force of less than 40,000 xg, preferably of less than or equal to 30,000 xg, more preferably of less than or equal to 20,000 xg and even more preferably of less than or equal to 15,000 xg and preferably of more than or equal to 1,000 xg, more preferably of more than or equal to 2,000 xg, even more preferably of more than or equal to 3,500 xg and most preferably of more than or equal to 5,000 xg.

Due to the crosslinking a net of high molecular weight is obtained which conveniently can be separated from the remaining liquid part of the sample by "standard" centrifugation, i.e. non-ultracentrifugation/centrifugation and force of less than 45,000 xg. Furthermore, no gradient centrifugation is necessary and hence preferably is excluded in the method of the present invention.

Independent from whether only one type of crosslinker is used or a mixture of crosslinkers each crosslinker comprises at least two funtional groups reactive with functional groups in the proteins of the sample which may be the same or different. These chemical groups preferably may be selected from the group comprising aryl azide, carbodiimide, carbonyl, diazirine, hydrazide, hydroxmethyl phosphine, imidoester, isocyanate, maleimide, *N*-hydroxysuccinyl (NHS) ester, pentafluorophenyl (PFP) ester, pyridyl disulfide and vinyl sulfone. Preferably the crosslinker may be a bifunctional crosslinker, i.e. a crosslinker comprising exactly two chemical groups reactive towards functional groups in the proteins present in the sample per molecule of crosslinker. Irrespective of whether the crosslinker is a bi- or a multifunctional crosslinker, preferably a crosslinker for reversibly crosslinking the virus particles is used, i.e. a crosslinker which either forms a covalent bond to functional groups in the proteins of the sample which may be cleaved and/or hydrolyzed when needed, such as e.g. imine bonds formed from aldehydes and amino groups or by using a crosslinker which comprises an internal cleavable bond, such as e.g. an internal disulfide bond, which may be cleaved by an appropriate cleaving agent without deteriorating the nucleic acids present in the sample. Preferably the crosslinker may be a bifunctional crosslinker for reversibly crosslinking the virus particles more preferably being selected from the group comprising bifunctional aldehydes, imidoesters and thio-cleavable crosslinkers comprising an internal disulfide bond, even more preferably being selected from the group comprising glutaraldehyde, paraformaldehyde, 3,3'-dithiobis(sulfosuccinimidyl proprionate) (DTSSP), sulfosuccinimidyl 6-(3'-[2-pyridyldithio]propionamido)hexanoate (sulfo-LC-SPDP), dimethyl suberimidate (DMS), and *N*-succinimidyl 3-(2-pyridyldithio)proprionate (SPDP).

Suitable groups reactive with amino groups in the side chains of proteins to be crosslinked include imido esters, NHS-esters, PFP-esters and carbodiamides, the latter also being reactive with carboxyl groups. Hyrazine groups in the target molecule may be crosslinked with the aid of carbonyl groups and a crosslinker, like maleiimides, pyridol disulfides and vinyl sulphones are reactive with sulfhydryl groups, the latter also being reactive with amines and hydroxyl groups. Isocyanates are reactive with hydroxyl groups as well.

The optimum ratio of the concentration of the crosslinker to the concentration of proteins present in the sample may vary with the type of crosslinker used, the sample, the concentration of the proteins in the sample, the concentration of further substances, including impurities, the pH of the sample and the like, which is well known to a person skilled in the art. In general it is preferred that the ratio of the concentration of the crosslinker to the concentration of proteins present in the sample may be greater than or equal 5:1, preferably greater than or equal to 10:1, even more preferably greater than or equal to 15:1 and most preferably greater than or equal to 20:1. The crosslinker (or the mixture of crosslinkers) may be added to the sample in substance, i.e. without any solvent, or as a solution in a suitable solvent not interfering in the crosslinking reaction. Such a solvent may e.g. comprise any of the liquids cited above or any suitable buffer, for instance, phosphate buffered saline (PBS) or another suitable phosohate buffer, Tris buffer, HEPES buffer or any other suitable buffer.

As already explained above, steps (ii) and (iii) may be carried out simultaneously, in a timely overlapping or alternating fashion, subsequently or step (iii) may be completely omitted. However, if present, step (iii) of incubating the mixture of the sample and the crosslinker may preferably be carried out at a temperature ranging of from -5 to 45 °C, preferably of from 0°to 37° C, even more preferably of from 0° to 30 °C and most preferably of from 0 °C to room temperature (20 ± 5 °C) for a time ranging from 5 s to 24 h, preferably of from 1 min to 12 h, even more preferably of from 5 min to 10 hours, even more preferably of from 10 min to 5 h and most preferably of from 15 min to 3.5 h.

As already explained above, preferably crosslinkers are used which introduce crosslinks to proteins present in the sample which can be essentially removed without deteriorating the nucleic acids present in the sample, in particular inside the virus particles, and preferably even without essentially deteriorating the virus particles. By using such "reversible" crosslinkers it is even possible to obtain free virus particles by essentially removing the crosslinks in the "net" after yielding the solid part of the sample according to step (v). The specific conditions for removing a crosslink or crosslinker depends on the type of crosslinker used which is well known to a person skilled in the art. However, most of the aforementioned crosslinks may be removed by acidic or alkaline hydrolysis in aqueous solution at a pH of either from 4 to 6.8 or 7.3 to 9, for instance crosslinks formed by amino bonds, or by the use of a reducing agent, preferably a dithiol, for instance crosslinks formed by crosslinkers having an internal disulfide bond.

As already explained above the essentially liquid sample of step (i) preferably may be obtained from an initial sample comprising animal cells and optionally at least one further type of cells, selected from microbial and plant cells or a combination thereof, by a method comprising the steps of:
a) essentially selectively lysing the animal cells substantially without lysing the further type of cells, if present, and without digesting the proteinaceous portion (capside) of the virus particles, preferably by contacting the initial sample with an essentially protease- and chaotrope-free lysis solution A, and
b) essentially removing any remaining cells and/or cell debris from the liquid portion of the sample, preferably by means of centrifuging, filtering and/or decanting.

The lysis solution A preferably may comprise at least one surfactant and optionally further at least one of
- a water soluble salt preferably being selected from the group comprising acetates, sulfates, glutamates or mixtures thereof as pH regulator,
   - a viscosity modifier, preferably being selected from the group comprising sugars, more preferably from the group comprising glucose and/or sucrose,
   - a polyanionic sulfonate, preferably sodium polyanethol sulfonate,
or a combination thereof. Particularly preferred the lysis solution A is essentially chaotrope-free.

Preferably the lysis solution A used in step a) may comprise at least one surfactant, a water soluble salt and/or a viscosity modifier, and optionally a polyanionic sulfonate, as described in detail in the following.

Preferably the lysis solution A furthermore may comprise at least one surfactant, more preferably at least one non-ionic surfactant. It may be preferred that all surfactants being present in the lysis solution A are non-ionic surfactants.

The non-ionic surfactants preferably may be selected from the group comprising sorbitan esters of fatty acids, polyoxyethylene sorbitan esters of fatty acids, polyoxyalkylene ethers of fatty alcohols, polyoxyalkylene ethers of alkylphenols, poloxamers, saponins or mixtures thereof.

Poloxamers are non-ionic triblock copolymers composed of an essential hydrophobic chain of polyoxypropylene flanked by two hydrophilic chains of polyoxyethylene and are commercially available for example from BASF (Ludwigshafen, Germany).

The lysis solution A preferably comprises at least one saponin. Saponins are amphipathic glycosids of steroids, steroid alkaloids or triterpenes. Being secondary metabolites, they can be isolated from natural sources, in particular fruits, roots, tubers, leafs and the like of plants, for example the fruits of *Sapindus* (soap nuts). Purified plant extracts comprising saponins are commercially available for example from Sigma-Aldrich (Saint Louis, MO, USA) or from AppliChem (Darmstadt, Germany).

It may be preferred that the lysis solution A comprises at least one saponin. It may be further preferred that any non-ionic surfactant being present in said lysis solution A represents a saponin.

The surfactant, preferably the saponin(s), may be present in said lysis solution A in a total amount of from about 0.01 to about 15% (w/v), based on the whole lysis solution A, preferably in an amount of from 0.1 to 14%, more preferably in an amount of from 1 to 13%, even more preferably of from about 2 to about 12%, even more preferably of from about 4 to 10% and most preferably of from about 5 to 8%. The mentioned amounts refer to the total amount of the included surfactants.

Furthermore, the lysis solution A may comprise a polyanionic sulfonate, preferably sodium polyanethol sulfonate. The polyanionic sulfonate may be present in a concentration of from 0.001 to 50 mM, preferably of from 0.01 to 10 mM, more preferably of from 0.1 to 5 mM, even more preferably of from 0.5 to 3 mM and most preferably of from 1.0 to 2.0 mM, based on the whole lysis solution A.

Furthermore, the lysis solution A may comprise a water-soluble salt which preferably may be selected from the group comprising water-soluble acetates, sulfates, glutamates or mixtures thereof, for example their sodium salts or a mixture thereof. Acetates and glutamates or a mixture thereof are particularly preferred. The water-soluble salt(s) preferably may be present in a total amount ranging of from 10 to 1.000 mM, preferably of from 50 to 850 mM, more preferably of from 100 to 500 mM and most preferably of from 160 to 250 mM, based on the whole lysis solution A.

Furthermore, the lysis solution A may comprise a viscosity modifier to increase or decrease the viscosity of the lysis solution A and optionally any solution to which the lysis solution A is added. The viscosity modifier preferably may be selected from the group comprising sugars, more preferably from the group comprising glucose and/or sucrose. The viscosity modifying agent preferably may be present in an amount ranging of from 10 to 1.000 mM, preferably of from 100 to 900 mM, more preferably of from 300 to 700 mM and most preferably of from 400 to 500 mM, based on the whole lysis solution A.

The pH of the lysis solution A preferably may be in the range of from 4 to 9, more preferably of from 4.5 to 8.5 and most preferably of from 5 to 8.

The lysis solution A preferably may comprise a combination of the aforementioned substances as well, and may for example represent an essentially chaotrop-free lysis solution A comprising at least one surfactant, preferably at least one saponin and either of i) a water-soluble salt and a viscosity modifier, ii) a polyanionic sulfonate and a viscosity modifier, iii) a polyanionic sulfonate and a water-soluble salt, or iv) a polyanionic sulfonate, a water-soluble salt and a viscosity modifier, all of the aforementioned substances preferably being selected from the groups already described in more detail above.

The lysis solution A may be added to the sample in an amount so that the ratio of the sample to the lysis solution A is 10:1 to 1:10, preferably 5:1 to 1: 5, further preferred 2:1 to 1:2, wherein a ratio of about 1:1 or exactly 1:1 is particularly preferred. The amount of lysis solution A to be added preferably is calculated dependent from the desired final concentration of the ingredients in the sample for lysis. Thus, if the lysis solution A comprises the above described ingredients in a concentration at the upper end as noted above, the ratio of lysis solution A : sample may be below 1, whereas said ratio increases above 1, when the lysis solution A concentration is more at the lower end of the ranges cited above for each of the ingredients. The preferred final concentration of the ingredients in the sample after addition of the lysis solution A is preferably represented by the half of the concentrations, respectively, as cited above or below for the lysis solution A.

By lysis of the animal cells on one hand said cells are selectively separated from the sample, on the other hand virus particles comprised inside of said cells are released by lysis, allowing the analysis of said virus particlesformerly comprised in the cells. After lysis of the animal cells, preferably in a further step, any animal nucleic acid, including both DNA and RNA, present in the sample may be essentially digested in the presence of the essentially intact virus particles before or after removing the remaining cells and/or cell debris from the liquid part of the sample according to step b), but before lysing the virus particles according to step vi). This preferably may be carried out enzymatically, for example by adding DNases and RNases to the sample, either simultaneously or subsequently. It may, however, be particularly preferred that both animal DNA as well as animal RNA are digested simultaneously by the action of one type of enzyme which is capable of digesting both DNA and RNA. Such types of enzyme is e.g. the endonucleasis commercially available under the trademark name Benzonase®, available by Merck, Germany, Cyanase™, available by RiboSolutions, USA or Pierce™ universal nuclease, available by Thermo Scientific, Germany, whithout being restricted to the mentioned. This step e.g. may be carried out after step (a), step (b), step (i), (ii), (iii), (iv) and (v) or simultaneously with any of steps (b) to (v), if appropriate. This step ensures that even small amounts of animal nucleic acids which may be associated with virus particles are efficiently digested before the vironucleic acids are released into solution from the capside. It is clear to a person skilled in the art that any nucleases present in the sample either have to be removed or inactivated before the vironucleic acids are released from their capsides, for example by the action of a protease.

In the method of the present invention the virus of the virus particles preferably comprises at least one human pathogen, and the animal cells comprise mammalian cells, preferably human cells, and/or the further cells, if present, preferably comprise microbial cells, preferably being selected from the group comprising bacterial, archae and fungi cells, including yeast cells, or a combination thereof, more preferably bacterial cells.

The present invention furthermore relates to the use of a crosslinker, preferably a crosslinker as described above, for concentrating, purifying and/or isolating virus particles from a sample, preferably in a method as described above.

The method furthermore relates to a kit for concentrating, purifying and/or isolating virus particles, comprising
a crosslinker for crosslinking at least a part of the proteinaceous portions of the virus particles either with each other and/or with any further protein being present in the sample, preferably a crosslinker as described above, and at least one of (A) an essentially protease- and chaotrope-free lysis solution A for essentially selectively lysing animal cells without lysing microbial and/or plant cells and virus particles, preferably a lysis solution A as described above, and (B) a lysis buffer B for lysing the virus particles to release their nucleic acids into solution, preferably as described above.

The kit preferably may further comprise at least one of the following components: means for purifying, concentrating and/or isolating viral and optionally microbial and/or plant nucleic acids, binding buffers, washing buffers, elution buffers, means for filtering and/or centrifuging the sample, DNases, RNases, endonucleases capable of digesting both, DNA and RNA, or a combination thereof. Further instructions for using the kit may be comprised in the kit or may be provided on the homepage of the supplier or any similar suitable place.

### List of Figures:

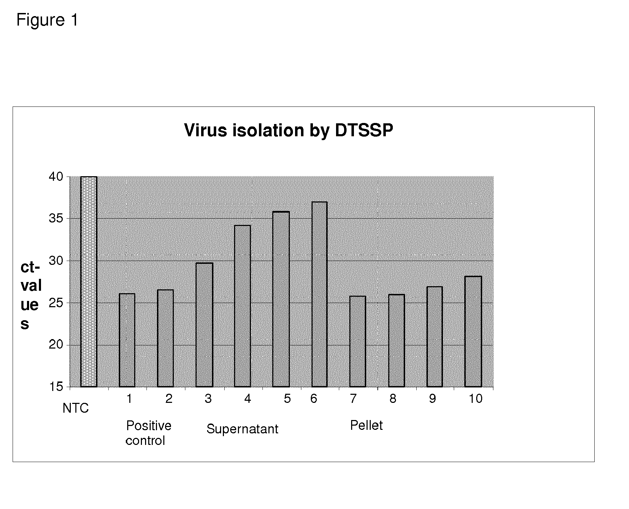

Fig. 1 shows ct values obtained from an RT-PCR of the supernatant and the pellet of a sample processed according to the method of the present invention (example 1). NTC: non target control, line 1 and 2: positive control: 1 µl virus without any treatment, line 3: supernatant after addition of 20-fold excess of DTSSP, line 4: supernatant after addition of 40-fold excess of DTSSP, line 5: supernatant after addition of 60-fold excess of DTSSP, line 6: supernatant after addition of 80-fold excess of DTSSP, line 7: pellet after addition of 20-fold excess of DTSSP, line 8: pellet after addition of 40-fold excess of DTSSP, line 9: pellet after addition of 60-fold excess of DTSSP, line 10: pellet after addition of 80-fold excess of DTSSP.

### Examples

### Example 1: Concentrating viral DNA in whole blood samples by crosslinking using DTSSP

To 667 µL citrate stabilized plasma, 333 µL phosphate buffer saline (PBS), 1 µL of virus-comprising (Baculovirus AcMNCV, about 10⁶/ml) PBS and DTSSP dithiobis(sulfosuccinimidyl propionate) (50%) in excess (20x, 40x, 60x, 80x) were added, mixed and left to stand on ice for 2 hours. The sample was then spinned for 10 minutes at room temperature at a force of 10,000 xg. The supernatant was discarded and 200 µL Tris pH 7,5 (50 mM) were added to the pellet. The resulting mixture was incubated for 15 minutes at room temperature. The solution obtained was further processed and purified using the QIAamp Min Elute Virus Spin Kit according to the manufacturers instructions and the eluate obtained was used in a RT-PCR.

The ct values obtained are shown in Figure 1. On the left "no target" control is shown which does not comprise any virus particles and virus DNA, respectively. In lanes 1 and 2 positive controls (1µL of Baculovirus AcMNCV, about 10⁶/ml in PBS) are shown. In lanes 3 to 6 the supernatant obtained after pelleting virus particles from the sample by the addition of a 20 (lane 3), 40 (lane 4), 60 (lane 5), and 80 (lane 6) excess of DTSSP to the sample is shown. In lanes 7 to 10 the pellet obtained from using said 20 (lane 7), 40 (lane 8), 60 (lane 9), and 80 (lane 10) excess, respectively, are shown. It can be seen that the viral concentration found in the supernatant decreases with an increasing amount of DTSSP, indicating the crosslinking and pelleting of the virus particles which are then removed by centrifugation. The moderate increase of ct values obtained from the pellets with increasing DTSSP excess is assigned to a dilution effect by the addition of DTSSP.

### Example 2: Isolating viral DNA from citrate stabilized blood samples using glutaraldehyde

1 mL of citrate stabilized blood spiked with 1 µL of a virus (Baculovirus AcMNCV, about 10⁶/ml in PBS) were spun in a centrifuge for 10 minutes at room temperature at a force of 10,000 xg. The plasma was collected and the pellet was discarded. The plasma was diluted by adding the same amount of buffer PBS and 10 µL of a commercially available benzonase® (Merck, Darmstadt, Germany) solution was added to the sample. The mixture was incubated for 30 minutes at room temperature in an end over end shaker. 20 µL of 50% aqueous solution of glutaraldehyde were then added and the mixture was spun in a centrifuge for 1 hour at room temperature at a force of 6,000 xg. The supernatant was collected and the pellet was purified using the QIAamp Min Elute Virus Spin Kit according to the manufacturers instructions. The eluate obtained was analyzed in a realtime PCR, while the supernatant was analyzed in an SDS page for controlling the amount of crosslinking. The ct value obtained from the sample is almost the same as that for the positive control (37,5 vs. 37,3). On a SDS page obtained from the supernatant no HSA can be detected in the sample treated according to the method of the present invention, as HSA has been crosslinked to the virus particles and hence pelleted in the centrifugation step.

## Claims

1. A method for concentrating, purifying and/or isolating virus particles from a sample comprising said virus particles and optionally non-viral proteins, comprising the steps of:
i) providing an essentially liquid sample comprising said virus particles,
ii) mixing said sample with at least one crosslinker to crosslink the virus particles with each other via the proteinaceous portion of the virus particles and/or to crosslink the virus particles with non-viral proteins, if present in the sample,
iii) optionally incubating the mixture obtained in step ii),
iv) essentially separating the crosslinked virus particles from the remaining liquid part of the sample.

2. The method according to claim 1, further comprising at least one of the following the steps:
v) essentially removing the crosslinks in the solid part of the sample obtained in step iv) to obtain free virus particles, and/or
vi) lysing the virus particles to release their nucleic acids into solution for further isolation, purification and/or analysis.

3. The method according to any of the preceding claims, wherein step iv) of essentially separating the solid part of the sample comprising crosslinked virus particles from the remaining liquid part is achieved by spinning the sample in a centrifuge at a force of less than 40,000 xg, preferably of less than or equal to 30,000 xg, more preferably of less than or equal to 20,000 xg and even more preferably of less than or equal to 15,000 xg and preferably of more than or equal to 1,000 xg, more preferably of more than or equal to 2,000 xg, even more preferably of more than or equal to 3,500 xg and most preferably of more than or equal to 5,000 xg.

4. The method according to any of the preceding claims, wherein the crosslinker comprises at least two funtional groups reactive towards functional groups in the proteins of the sample, which may be the same or different and are selected from the group comprising aryl azide, carbodiimide, carbonyl, diazirine, hydrazide, hydroxmethyl phosphine, imidoester, isocyanate, maleimide, *N*-hydroxysuccinyl (NHS) ester, pentafluorophenyl (PFP) ester, pyridyl disulfide and vinyl sulfone, the crosslinker preferably being a bifunctional crosslinker for reversibly crosslinking the virus particles, more preferably being selected from the group comprising bifunctional aldehydes, imidoesters and thio-cleavable crosslinkers comprising an internal disulfide bond, even more preferably being selected from the group comprising glutaraldehyde, paraformaldehyde, 3,3'-dithiobis(sulfosuccinimidyl proprionate) (DTSSP), sulfosuccinimidyl 6-(3'-[2-pyridyldithio]propionamido)hexanoate (sulfo-LC-SPDP), dimethyl suberimidate (DMS), and *N*-succinimidyl 3-(2-pyridyldithio)proprionate (SPDP).

5. The method according to any of the preceding claims, wherein the ratio of the concentration of the crosslinker to the concentration of proteins in Mol/L present in the sample is greater than or equal 5:1, preferably greater than or equal to 10:1, even more preferably greater than or equal to 15:1 and most preferably greater than or equal to 20:1.

6. The method according to any of the preceding claims, wherein step iii) of incubating the mixture of the sample and the crosslinker is carried out at a temperature ranging of from -5 to 45 °C, preferably of from 0 to 37 °C, even more preferably of from 0 to 30 °C and most preferably of from 0 °C to room temperature (20 ± 5 °C) for a time ranging from 5 s to 24 h, preferably of from 1 min to 12 h, even more preferably of from 5 min to 10 hours, even more preferably of from 10 min to 5 h and most preferably of from 15 min to 3.5 h.

7. The method according to any of claims 2 to 6, wherein the crosslinks are removed in step v) by acidic or alkaline hydrolysis in aqueous solution at a pH of either from 4 to 6.8 or 7.3 to 9 or by a reducing agent, preferably a dithiol.

8. The method according to any of the preceding claims, wherein the sample comprises or is derived from tissue, tissue homogenates, cell cultures, bone marrow aspirates, bone homogenates, swabs, swab rinsates or body fluids, preferably body fluids, more preferably body fluids being selected from the group comprising amniotic fluid, aqueous humor, bile, bladder lavage, blood, breast exudates, bronchoalveolar lavage, cerebrospinal fluid, chyle, chyme, cytosol, feces, gastric contents, interstitial fluid, joint fluid, lymph, menses, mucus, plasma, pleural fluid, pus, saliva, sebum, semen, serum, sputum, sweat, synovial fluid, tears, urine, vaginal secretions and vitreous humour.

9. The method according to any of the preceding claims, wherein the essentially liquid sample of step i) is obtained from an initial sample, comprising besides the virus particles mammal cells and optionally at least one further type of cells, selected from microbial and plant cells or a combination thereof, by a method comprising the steps of:
a) essentially selectively lysing the mammal cells substantially without lysing the further type of cells, if present, and without digesting the proteinaceous portion (capside) of the virus particles, preferably by contacting the initial sample with an essentially protease- and chaotrope-free lysis solution A, and
b) essentially removing any remaining cells and/or cell debris from the liquid part of the sample, preferably by means of centrifuging, filtering and/or decanting.

10. The method acoording to claim 9, wherein the essentially chaotrope-free lysis solution A comprises at least one nonionic surfactant, preferably being selected from the group comprising sorbitan esters of fatty acids, polyoxyethylene sorbitan esters of fatty acids, polyoxyalkylene ethers of fatty alcohols, polyoxyalkylene ethers of alkylphenols, poloxamers, saponins or mixtures thereof, more preferably comprising saponin, the nonionic surfactant(s) preferably being present in an amount of from 0.1 to 15 wt% more preferably in an amount of from 1 to 10 wt%, even more preferably in an amount of from 2 to 8 wt% and most preferably in an amount of from 3 to 5 wt%, based on the lysis solution A, and optionally further at least one of
- a water soluble soluble salt, preferably being selected from the group comprising acetates, sulfates, glutamates or mixtures thereof,
- a viscosity modifier, preferably being selected from the group comprising sugars, more preferably from the group comprising glucose and/or sucrose,
- a polyanionic sulfonate, preferably sodium polyanethol sulfonate,
or a combination thereof.

11. The method according to claim 9 or 10, further comprising a step of essentially digesting any animal nucleic acids, including both, DNA and RNA, present in the sample in the presence of the essentially intact virus particles before or after removing the remaining cells and/or cell debris from the liquid part of the sample according to step b), but before lysing the virus particles according to step vi).

12. The method according to any of the preceding claims, wherein the virus comprises at least one human pathogen, and wherein the animal cells, if present, comprise mammalian cells, preferably human cells, and/or the further cells, if present, comprise microbial cells, preferably being selected from the group comprising bacterial, archae and fungi cells, including yeast cells, or a combination thereof, more preferably bacterial cells.

13. Use of a crosslinker, preferably a crosslinker as described in claim 4, for concentrating, purifying and/or isolating virus particles from a sample, preferably in a method as described in any of the preceding claims.

14. A kit for concentrating, purifying and/or isolating virus particles, comprising a crosslinker for crosslinking at least a part of the proteinaceous portions of the virus particles either with each other and/or with any further protein being present in the sample, preferably a crosslinker as described in claim 4, and at least one of an essentially protease- and chaotrope-free lysis solution A for essentially selectively lysing animal cells without lysing microbial and/or plant cells and virus particles, preferably a lysis solution A as described in claim 10, and a lysis buffer B for lysing the virus particles to release their nucleic acids into solution.

15. The kit according to claim 14, further comprising at least one of the following components: instructions for using the kit, means for purifying, concentrating and/or isolating viral and optionally microbial and/or plant nucleic acids, binding buffers, washing buffers, elution buffers, means for filtering and/or centrifuging the sample, DNases, RNases, endonucleases capable of digesting both, DNA and RNA, or a combination thereof.
